# EUROPEAN PATENT APPLICATION

(11) **EP 4 424 168 A1**
(43) Date of publication of application: **04.09.2024**
(21) Application number: 22887486.3
(22) Date of filing: 20.10.2022
(51) Int. Cl.: A01N 63/20, A61K 35/747, A61P 1/02, A23L 33/135, C12R 1/23

(54) **ANTIBACTERIAL COMPOSITION CONTAINING LACTOBACILLUS STRAIN CULTURE FILTRATE**

(30) Priority: 29.10.2021 KR 20210147058
(71) Applicant: Cell Biotech Co., Ltd., Wolgot-myeon Gyeonggi-do 10003 (KR)
(72) Inventor: CHUNG, Myung Jun, Seoul 06622 (KR)
(74) Representative: Patentanwaltskanzlei Matschnig & Forsthuber OG
(86) International application number: PCT/KR2022/016012
(87) International publication number: WO 2023/075290

(57) **Abstract**

The present invention provides an antibacterial composition comprising an LP3 culture filtrate obtained by culturing a *Lactobacillus plantarum* in a first medium containing yeast extract and peptone as a nitrogen source(N-source) without containing beef extract, wherein the antibacterial composition has an antibacterial activity against a Streptococcus mutans strain.

The composition provided by the present invention has an antibacterial activity that effectively kills various harmful bacteria such as the causative bacteria of periodontitis, so that it can be used as an antibacterial agent for various purposes.

## Description

### Technical Field

The present invention relates to an antibacterial composition, and more particularly, to an antibacterial composition comprising a culture filtrate of a Lactobacillus-based strain capable of effectively killing harmful bacteria such as the causative bacteria of periodontitis.

### Background Art

Recently, the number of patients with periodontal disease is increasing due to decreased immunity and increased nutritional and metabolic disorders caused by wrong eating habits and aging. The direct cause of periodontal disease includes a bacterial film called plaque, which is continuously formed on the teeth, and tartar formed when the plaque is hardened without being removed. When the plaque and the tartar build up, the gums separate from the teeth, causing inflammation between the teeth and the gums.

Periodontal disease is divided into gingivitis and periodontitis according to the degree of inflammation. Gingivitis may cause red, swollen gums and bleeding symptoms, but it is possible to recover to some extent from gingivitis with careful brushing. Periodontitis is a more advanced inflammation of gingivitis, and in the case of periodontitis, bad breath occurs, pus comes out between the teeth and the gums, and discomfort is felt during chewing. In addition, teeth loosening may occur.

Here, a Streptococcus mutans is a representative oral bacterium and is a type of streptococcus. The Streptococcus mutans has been pointed out as the main cause of tooth decay along with Lactobacillus, and as far as is known, it discolors teeth black and causes them to decay.

On the other hand, health functional foods and therapeutic agents having the effect of preventing or treating periodontal disease are being developed. In particular, it has recently been reported that lactic acid bacteria are effective in preventing periodontal disease by inhibiting periodontal disease-causing bacteria in the oral cavity (Korean Registered Patent No. 10-1690090), and strains showing excellent antibiotic activity against periodontal disease bacteria have been developed.

Therefore, the present inventors found that the culture filtrate obtained by culturing a *Lactobacillus plantarum* and a *Lactobacillus acidophilus,* known to be effective in treating periodontitis, in a medium with an optimized content of a nitrogen source (N-source) showed excellent antibacterial activity against the Streptococcus mutans than the culture filtrate obtained by culturing the *Lactobacillus plantarum* and the *Lactobacillus acidophilus* in a MRS medium, so that the inventors completed the present invention.

### Disclosure

### Technical Problem

One object of the present invention is to provide an antibacterial composition having excellent antibacterial activity against various harmful bacteria such as the causative agent of periodontitis.

Another object of the present invention is to provide a pharmaceutical composition for preventing or treating periodontitis comprising the antibacterial composition as an active ingredient.

Still another object of the present invention is to provide a food composition for preventing or treating periodontitis comprising the antibacterial composition as an active ingredient.

However, objects which are to be achieved by the present invention are not limited to the above-mentioned objects, and other objects of the present invention will be clearly understood by those skilled in the art from the following description.

### Technical Solution

One embodiment of the present invention relates to an antibacterial composition comprising an LP3 culture filtrate obtained by culturing a *Lactobacillus plantarum* in a first medium containing yeast extract and peptone as a nitrogen source(N-source) without containing beef extract, wherein the antibacterial composition has an antibacterial activity against a Streptococcus mutans strain.

The present invention may be characterized in that a content ratio of the nitrogen source and a carbon source in the first medium is 0.3 to 1: 1.

The present invention may be characterized in that a content ratio of the yeast extract and the peptone in the first medium is 0.5 to 4: 1.

The present invention may be characterized in that the nitrogen source is contained in 20 to 35 % by weight based on the total weight of the first medium.

The present invention may be characterized in that the first medium contains glucose as the carbon source.

The present invention may be characterized in that the first medium contains 2 to 4 % by weight of dibasic potassium phosphate, 8 to 10 % by weight of sodium acetate trihydrate, 0.01 to 1 % by weight of magnesium sulfate heptahydrate, 0.01 to 1 % by weight of manganese sulfate tetrahydrate, 3 to 5 % by weight of triammonium citrate, and 1 to 3 % by weight of polysorbate 80, based on a total weight of the first medium.

The present invention may be characterized in that the *Lactobacillus plantarum* is *Lactobacillus plantarum* LP3 (Accession Number: KCTC 10782BP).

The present invention may be characterized in that the culturing is performed for 6 to 48 hours at a temperature of 30 to 40 °C.

The present invention may be characterized in that the antibacterial composition comprises a LA1 culture filtrate obtained by culturing an *Lactobacillus acidophilus* in a second medium containing the yeast extract and isolated soybean protein as the nitrogen source without containing the beef extract.

The present invention may be characterized in that a content ratio of the nitrogen source and a carbon source in the second medium is 0.5 to 1.5: 1.

The present invention may be characterized in that the content of the yeast extract and the isolated soybean protein in the second medium is 1 to 10: 1.

The present invention may be characterized in that the nitrogen source is contained in 30 to 60 % by weight based on the total weight of the second medium.

The present invention may be characterized in that the second medium contains glucose as the carbon source.

The present invention may be characterized in that the second medium contains 2 to 4 % by weight of dibasic potassium phosphate, 5 to 10 % by weight of sodium acetate trihydrate, 0.01 to 1 % by weight of magnesium sulfate heptahydrate, 0.01 to 1 % by weight of manganese sulfate tetrahydrate, 2 to 5 % by weight of triammonium citrate, and 1 to 3 % by weight of polysorbate 80, based on the total weight of the second medium.

The present invention may be characterized in that the *Lactobacillus acidophilus* is a *Lactobacillus acidophilus* LA1 (Accession Number: KCTC 11906BP).

The present invention may be characterized in that the culturing is performed for 6 to 48 hours at a temperature of 30 to 40 °C.

The present invention may be characterized in that after culturing the LP3 culture filtrate and the LA1 culture filtrate, a bacterial cell is removed by at least one process of centrifugation and membrane separation.

The antibacterial composition of the present invention may be characterized in that it comprises one or more selected from the group consisting of propolis, citron extract, bamboo salt extract, coconut extract, spearmint extract, sage extract, green tea extract, grapefruit extract, aloe extract, licorice extract, chamomile extract, plum extract, red ginseng extract, Ulmi cortex extract, cinnamon extract, tea tree extract, lemon extract, and grapefruit seed extract.

Another embodiment of the present invention relates to a pharmaceutical composition for preventing or treating periodontitis comprising the antibacterial as an active ingredient.

Still another embodiment of the present invention relates to a food composition for preventing or treating periodontitis comprising the antibacterial composition as an active ingredient.

### Advantageous Effects

The composition provided by the present invention has an antibacterial activity that effectively kills various harmful bacteria such as the causative bacteria of periodontitis, so that it can be used as an antibacterial agent for various purposes.

In addition, the composition provided by the present invention can prevent, alleviate or treat periodontitis by effectively killing a *Streptococcus mutans,* a representative causative agent of periodontitis.

### Description of Drawings

FIG. 1 shows the antibacterial activity of the culture filtrate of a Lactobacillus-based strain against a *Streptococcus mutans.*
FIG. 2 shows the antibacterial activity of an LP3 culture filtrate and an LA1 culture filtrate against a *Streptococcus mutans.*

### Mode for Invention

The term "periodontitis" in the present invention means that plaque and tartar are accumulated in a V-shaped narrow sulcus called a gingival sulcus between the teeth and the gums (gingiva), causing the gums to lift from the teeth, and bacteria attack the area below the gum line in this sulcus, damaging the periodontal ligament and surrounding bone tissue.

The term "culture filtrate" in the present invention means a liquid from which bacterial cells existing in the supernatant of the precipitate of the culture solution obtained by culturing a strain in a medium are removed.

As a result of diligent efforts by the inventors of the present invention to develop an antibacterial composition, the inventors found that the LP3 culture filtrate obtained by culturing a *Lactobacillus plantarum* in a first medium containing yeast extract and peptone as a nitrogen source (N-source) but not containing beef extract significantly increased the antibacterial effect against a *Streptococcus mutans,* the causative agent of periodontitis, and then completed the invention.

One embodiment of the present invention relates to an antibacterial composition comprising an LP3 culture filtrate obtained by culturing a *Lactobacillus plantarum* in a first medium containing yeast extract and peptone as a nitrogen source(N-source) without containing beef extract, and having an antibacterial activity against a *Streptococcus mutans* strain.

The LP3 culture filtrate may be a filtrate obtained by filtering and removing bacterial cells from a culture medium in which microorganisms are cultured. More specifically, the LP3 culture filtrate may be obtained by centrifuging the culture medium and filtering the culture medium through a filter paper, but the LP3 culture filtrate is not limited thereto.

The LP3 culture filtrate has a remarkable antibacterial activity against the *Streptococcus mutans* compared to the culture medium obtained by culturing the *Lactobacillus plantarum* in the first medium. Since the *Streptococcus mutans* is a type of lactic acid bacteria, it has no antibacterial activity by Lactobacillus, and shows little inhibition of organic acids produced by the Lactobacillus at pH 4 or higher. However, since the LP3 culture filtrate shows antibacterial activity in such an environment, it can be seen that the antibacterial activity effect by the culture filtrate, which is a non-strain metabolite, is remarkable, compared to the LP3 strain itself.

The first medium in the present invention does not contain the beef extract as a medium component when culturing the *Lactobacillus plantarum,* so that the culture filtrate can have a remarkable effect on antibacterial activity. When the beef extract is added to the first medium in the present invention, it is difficult to expect an additional improved effect of antibacterial activity.

In the present invention, the content ratio of the nitrogen source and the carbon source in the first medium may be 0.3 to 1: 1, preferably 0.5 to 0.7: 1. The culture filtrate of *Lactobacillus plantarum* cultured in the first medium in which the content ratio of the nitrogen source and the carbon source is optimized can obtain an improved effect of antibacterial activity against *Streptococcus mutans.*

In the present invention, the content ratio of the yeast extract and the peptone in the first medium may be 0.5 to 4: 1, preferably 1 to 3: 1.

In the present invention, the nitrogen source may be contained in 20 to 35 % by weight, preferably 25 to 28 % by weight based on the total weight of the first medium.

In the present invention, the first medium may contain sugars such as glucose, sucrose, fructose, maltose, lactose and starch, organic acids such as malic acid and citric acid, and fatty acids with low molecular weight. Preferably, glucose may be contained.

In the present invention, the first medium may contain 2 to 4 % by weight of dibasic potassium phosphate, 8 to 10 % by weight of sodium acetate trihydrate, 0.01 to 1 % by weight of magnesium sulfate heptahydrate, 0.01 to 1 % by weight of manganese sulfate tetrahydrate, 3 to 5 % by weight of triammonium citrate, and 1 to 3 % by weight of polysorbate 80, based on the total weight of the first medium.

In the present invention, the *Lactobacillus plantarum* may be *Lactobacillus plantarum* LP3 (Accession Number: KCTC 10782BP).

In the present invention, the culture conditions of the *Lactobacillus plantarum* are not particularly limited. For example, the *Lactobacillus plantarum* may be cultured for 6 to 48 hours at a temperature of 30 to 40 °C, and the culture condition may be appropriately adjusted according to the culture scale.

In the present invention, the antibacterial composition may comprise a LA1 culture filtrate obtained by culturing an *Lactobacillus acidophilus* in a second medium containing the yeast extract and isolated soybean protein as the nitrogen source without containing the beef extract.

The LA1 culture filtrate refers to a filtrate obtained by filtering and removing bacterial cells from a culture medium in which microorganisms are cultured. More specifically, the LA1 culture filtrate may be obtained by centrifuging the culture medium and filtering the culture medium through a filter paper, but is not limited thereto.

The LA1 culture filtrate has a remarkable antibacterial activity against the *Streptococcus mutans* compared to the culture medium obtained by culturing the *Lactobacillus acidophilus* in the second medium. Since the *Streptococcus mutans* is a type of lactic acid bacteria, it has no antibacterial activity by Lactobacillus, and shows little inhibition of organic acids produced by the Lactobacillus at pH 4 or higher. However, since the LA1 culture filtrate shows antibacterial activity in such an environment, it can be seen that the antibacterial activity effect by the culture filtrate, which is a non-strain metabolite, is remarkable, compared to the LA1 strain itself.

The second medium in the present invention does not contain the beef extract when culturing the *Lactobacillus acidophilus,* so that an unexpected improvement in the antibacterial activity of the LA1 culture filtrate can be obtained. When the beef extract is added to the second medium in the present invention, it is difficult to expect an additional improved effect of antibacterial activity.

In the present invention, a content ratio of the nitrogen source and a carbon source in the second medium may be 0.5 to 1.5: 1, preferably 1.17: 1. The LA1 culture filtrate cultured in the second medium in which the content ratio of the nitrogen source and the carbon source is optimized can obtain an improved effect of antibacterial activity against *Streptococcus mutans.*

In the present invention, the content of the yeast extract and the isolated soybean protein in the second medium may be 1 to 10: 1, preferably 6: 1.

In the present invention, the nitrogen source may be contained in 30 to 60 % by weight, preferably 40 to 50 % by weight based on the total weight of the second medium.

In the present invention, the second medium may contain sugars such as glucose, sucrose, fructose, maltose, lactose and starch, organic acids such as malic acid and citric acid, and fatty acids with low molecular weight. Preferably, glucose may be contained.

In the present invention, the second medium may contain 2 to 4 % by weight of dibasic potassium phosphate, 5 to 10 % by weight of sodium acetate trihydrate, 0.01 to 1 % by weight of magnesium sulfate heptahydrate, 0.01 to 1 % by weight of manganese sulfate tetrahydrate, 2 to 5 % by weight of triammonium citrate, and 1 to 3 % by weight of polysorbate 80, based on the total weight of the second medium.

In the present invention, the *Lactobacillus acidophilus* may be a *Lactobacillus acidophilus* LA1 (Accession Number: KCTC 11906BP).

In the present invention, the culture conditions of the *Lactobacillus acidophilus* are not particularly limited. For example, the *Lactobacillus acidophilus* may be cultured for 6 to 48 hours at a temperature of 30 to 40 °C, and the culture condition may be appropriately adjusted according to the culture scale.

The antibacterial composition of the present invention may comprise an oral antibacterial substance, one or more selected from the group consisting of propolis, citron extract, bamboo salt extract, coconut extract, spearmint extract, sage extract, green tea extract, grapefruit extract, aloe extract, licorice extract, chamomile extract, plum extract, red ginseng extract, Ulmi cortex extract, cinnamon extract, tea tree extract, lemon extract, and grapefruit seed extract.

Another embodiment of the present invention relates to a pharmaceutical composition for preventing or treating periodontitis comprising the antibacterial composition as an active ingredient.

In the present invention, the term "preventing" may refer to all actions that block symptoms of periodontitis or inhibit or delay the symptoms by use of the pharmaceutical composition of the present invention.

In addition, in the present invention, the term "treating" may refer to all actions that alleviate or beneficially change symptoms of periodontitis by use of the pharmaceutical composition of the present invention.

In the present invention, the pharmaceutical composition may be in the form of capsules, tablets, granules, injectable solutions, ointments, powders or beverages, and the pharmaceutical composition may be for use in humans.

For use, the pharmaceutical composition of the present invention may be formulated into oral preparations such as powders, granules, capsules, tablets, aqueous suspensions and the like, and forms such as external preparations, suppositories and sterile injectable solutions, according to commonly used methods, but is not limited thereto. The pharmaceutical composition of the present invention may contain a pharmaceutically acceptable carrier. For oral administration, the pharmaceutically acceptable carrier may include a binder, a lubricant, a disintegrant, an excipient, a solubilizer, a dispersing agent, a stabilizer, a suspending agent, a coloring agent, a flavoring agent and the like. For injectable preparations, the pharmaceutically acceptable carrier may include a buffering agent, a preservative, an analgesic, a solubilizer, an isotonic agent, a stabilizer and the like. For topical administration, the pharmaceutically acceptable carrier may include a base, an excipient, a lubricant, a preservative and the like. The pharmaceutical composition of the present invention may be formulated into a variety of dosage forms in combination with the aforementioned pharmaceutically acceptable carriers. For example, for oral administration, the pharmaceutical composition may be formulated into tablets, troches, capsules, elixirs, suspensions, syrups, wafers or the like. For injectable administration, the pharmaceutical composition may be formulated as a unit dosage ampoule or a multiple dosage form. In addition, the pharmaceutical composition may also be formulated into solutions, suspensions, tablets, capsules and sustained-release preparations.

Meanwhile, examples of the carrier, excipient, and diluent suitable for the formulation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, mineral oil or the like. In addition, the pharmaceutical composition of the present invention may further contain a filler, an anti-agglutinating agent, a lubricating agent, a wetting agent, a flavoring agent, an emulsifying agent, a preservative or the like.

Routes of administration of the pharmaceutical composition according to the present invention include, but are not limited to, oral, intravenous, intramuscular, intra-arterial, intramarrow, intrathecal, intracardiac, transdermal, subcutaneous, intraperitoneal, intranasal, intraintestinal, topical, sublingual or rectal routes. Oral or parenteral administration is preferred.

As used herein, the term "parenteral" includes subcutaneous, intracutaneous, intravenous, intramuscular, intraarticular, intrasynovial, intrasternal, intrathecal, intralesional, and intracranial injection or infusion techniques. The pharmaceutical composition of the present invention may also be administered in the form of suppositories for rectal administration.

The pharmaceutical composition of the present invention may vary depending on various factors, including the activity of a particular compound used, the subject's age, weight, general health, sex, diet, administration time, administration route, excretion rate, drug combination and the severity of a particular disease to be prevented or treated. The dose of the pharmaceutical composition may be suitably selected by a person skilled in the art depending on the patient's condition, weight, the severity of the disease, the type of drug, administration route and period, and the pharmaceutical composition may be administered at a dose of 0.0001 to 50 mg/kg or 0.001 to 50 mg/kg per day. The pharmaceutical composition may be administered once or several times per day. The dose does not limit the scope of the present invention in any way. The pharmaceutical composition according to the present invention may be formulated as pills, coated tablets, capsules, liquids, gels, syrups, slurries or suspensions.

Still another embodiment of the present invention relates to a food composition for preventing or treating periodontitis comprising the antibacterial composition as an active ingredient.

In the present invention, the term "preventing" may refer to all actions that block symptoms of periodontitis or inhibit or delay the symptoms by use of the food composition of the present invention.

In addition, In addition, in the present invention, the term "treating" may refer to all actions that alleviate or beneficially change symptoms of periodontitis by use of the food composition of the present invention.

The food composition of the present invention may be prepared as various foods, for example, beverages, gums, teas, vitamin complexes, powders, granules, tablets, capsules, confectionery, cakes, bread and the like. The food composition of the present invention is based on a plant extract having little or no toxicity and side effects, and thus may be used without anxiety for preventive purposes over a long period of time.

When the culture filtrate of the present invention is comprised in a food composition according to the present invention, it may be added in an amount of 0.1 to 70 % by weight based on the total weight of the composition.

Here, when the food composition is prepared as a beverage, there is no particular limitation, except that the beverage contains the food composition at the indicated percentage. The beverage may additionally contain various flavorings or natural carbohydrates, like conventional beverages. Examples of the natural carbohydrates include monosaccharides such as glucose, disaccharides such as fructose, polysaccharides such as sucrose, conventional sugars such as dextrin, cyclodextrin or the like, and sugar alcohols such as xylitol, sorbitol, erythritol or the like. Examples of the flavorings include natural flavorings (thaumatin, stevia extracts, such as rebaudioside A, glycyrrhizin, etc.) and synthetic flavorings (saccharin, aspartame, etc.).

In addition, the food composition of the present invention may contain various nutrients, vitamins, minerals (electrolytes), flavorings such as synthetic flavorings and natural flavorings, colorants, pectic acid and its salt, alginic acid and its salt, organic acids, protective colloidal thickeners, pH adjusting agents, stabilizers, preservatives, glycerin, alcohol, carbonizing agents as used in carbonated beverages, etc.

Such components may be used individually or in combination. Although the percentage of such additives is not of great importance, it is generally selected in the range of 0.1 to about 70 parts by weight based on 100 parts by weight of the food composition of the present invention.

Hereinafter, the present invention will be described in more detail through examples. These examples are only for explaining the present invention in more detail, and it will be apparent to those skilled in the art that the scope of the present invention is not limited by these examples according to the gist of the present invention.

### Examples

### [Preparation Example 1] Preparation of culture filtrate of Lactobacillus plantarum cultured in MRS medium

A *Lactobacillus plantarum* CBT-LP3 (Accession No. KCTC 10782BP) was inoculated at 1 % by weight in 0.1 L of MRS medium containing the ingredients shown in Table 1 below, and then cultured at 37 °C for 16 hours. Thereafter, the entire culture medium was centrifuged (10,000 Xg, 10 minutes) to prepare a culture filtrate.

### [Preparation Example 21 Preparation of culture filtrate of Lactobacillus acidophilus cultured in MRS medium

A *Lactobacillus acidophilus* CBT-LA1 (Accession number: KCTC 11906BP) was prepared as a culture filtrate in the same manner as in Preparation Example 1.

### [Preparation Example 3] Preparation of culture filtrate of Lactobacillus casei cultured in MRS medium

*A Lactobacillus casei* CBT-LC5 (Accession number: KCTC 12398BP) was prepared as a culture filtrate in the same manner as in Preparation Example 1.

### [Preparation Example 4] Preparation of culture filtrate of Lactobacillus paracasei cultured in MRS medium

A *Lactobacillus paracasei* CBT-LPC5 (Accession number: KCTC 12451BP) was prepared as a culture filtrate in the same manner as in Preparation Example 1.

### [Preparation Example 5] Preparation of culture filtrate of Lactobacillus gasseri cultured in MRS medium

A *Lactobacillus gasseri* CBT-LGA1 (Accession number: KCTC 12936BP) was prepared as a culture filtrate in the same manner as in Preparation Example 1.

**[Table 1]**

| Ingredient of MRS medium | | Content(wt%) |
|---|---|---|
| Carbon source | Glucose | 36.3 |
| Nitrogen source | Peptone | 18.1 |
| | Isolated soybean protein | - |
| | Yest extract | 9.1 |
| | Beef extract | 18.1 |
| Others | Dibasic potassium phosphate | 3.6 |
| | Sodium acetate trihydrate | 9.1 |
| | Magnesium sulfate heptahydrate | 0.2 |
| | Manganese sulfate tetrahydrate | 0.1 |
| | Triammonium citrate | 3.6 |
| | Polysorbate 80 | 1.8 |

### [Experimental Example 11 Strain screening

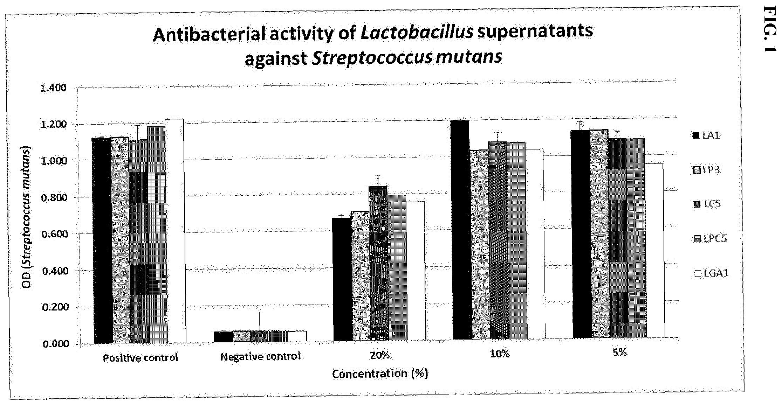

After culturing a test strain, a *Streptococcus mutans,* in a 96-well plate (Round type) with Brain heart infusion medium at 37 °C for about 24 hours, cells were prepared with an optical density(O.D) of 0.5 McFarland standard and a suspension was prepared so that a final strain concentration was 5.0 × 10⁵ CFU/ml. The 2-fold dilution of the culture filtrate prepared in Preparation Examples 1 to 5 and the suspension medium in which the test strain was grown were mixed and cultured at a ratio of 1:1. Then, the Microplate Reader O.D value (600 nm) was measured to determine a degree of growth inhibition of the *Streptococcus mutans* and the result was shown in FIG. 1. In the antibacterial activity test of the *Streptococcus mutans,* the higher the O.D value, the more the *Streptococcus mutans* is present in the medium.

As shown in FIG. 1, the O.D measured when the *Streptococcus mutans* suspension was mixed with each of the culture filtrates of Preparation Examples 1 and 2 was lower than the O.D measured when the Streptococcus mutans suspension was mixed with each of the culture filtrates of Preparation Examples 3 to 5. As a result, it can be seen that the culture filtrates of the *Lactobacillus plantarum* and *Lactobacillus acidophilus* strains have higher antibacterial activity against the *Streptococcus mutans* compared to the culture filtrates of the *Lactobacillus casei, Lactobacillusparacasei* and *Lactobacillus gasseri* strains.

### [Preparation Example 61 Preparation of LP3 culture filtrate

A *Lactobacillus plantarum* CBT-LP3 (Accession No. KCTC 10782BP) was inoculated at 1 % by weight in 0.1 L of the first medium containing the ingredients shown in Table 2 below, and then cultured at 37 °C for 16 hours. Thereafter, the entire culture medium was centrifuged (10,000 Xg, 10 minutes) to prepare the LP3 culture filtrate.

### [Preparation Example 7] Preparation of LA1 culture filtrate

A *Lactobacillus acidophilus* CBT-LA1 (Accession No. KCTC 11906BP) was inoculated at 1 % by weight in 0.1 L of the second medium containing the ingredients shown in Table 2 below, and then cultured at 37 °C for 16 hours. Thereafter, the entire culture medium was centrifuged (10,000 Xg, 10 minutes) to prepare the LA1 culture filtrate.

**[Table 2]**

| Medium ingredient | | First medium | Second medium |
|---|---|---|---|
| | | Content(wt%) | Content(wt%) |
| Carbon source | Glucose | 54.4 | 39.9 |
| Nitrogen source | Peptone | 9.1 | - |
| | Isolated soybean protein | - | 6.6 |
| | Yeast extract | 18.1 | 39.9 |
| | Beef extract | - | - |
| Others | Dibasic potassium phosphate | 3.6 | 2.7 |
| | Sodium acetate trihydrate | 9.1 | 6.7 |
| | Magnesium sulfate heptahydrate | 0.2 | 0.1 |
| | Manganese sulfate tetrahydrate | 0.1 | 0.1 |
| | Triammonium citrate | 3.6 | 2.7 |
| | Polysorbate 80 | 1.8 | 1.3 |

### [Experimental Example 21 Evaluation of antibacterial effect of culture filtrate

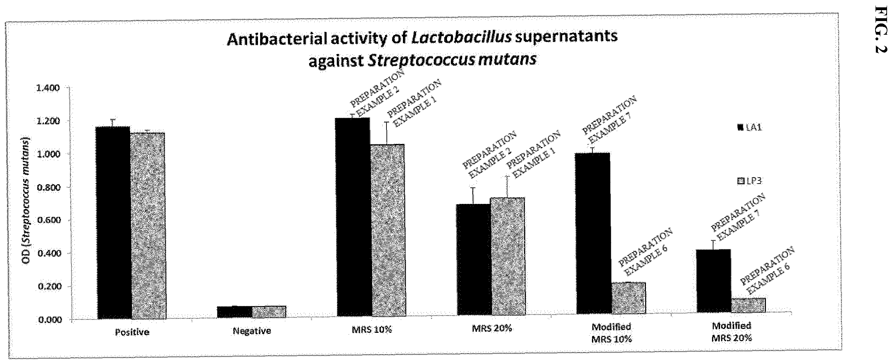

For the culture filtrate obtained in Preparation Examples 6 and 7, the experiment was performed in the same manner as in Experimental Example 1, and the result was shown in FIG. 2 in comparison with the experimental results for Preparation Examples 1 and 2 by Experimental Example 1.

As shown in FIG. 2, it was confirmed that the absorbance measured when the *Streptococcus mutans* suspension was mixed with the culture filtrates of Preparation Examples 6 and 7 in which the *Lactobacillus plantarum* and the *Lactobacillus acidophilus* were cultured in the medium containing an optimized content ratio of nitrogen source and carbon source without the beef extract, respectively, was significantly lower than the absorbance measured when the *Streptococcus mutans* suspension was mixed with the culture filtrates of Preparation Examples 1 and 2 in which the *Lactobacillus plantarum* and the *Lactobacillus acidophilus* were cultured in the MRS medium containing the beef extract.

Therefore, it was confirmed that the LP3 culture filtrate and LA1 culture filtrate, respectively, obtained by culturing in the first medium and the second medium in which the beef extract was excluded and the content ratio of the nitrogen source and the carbon source was optimized killed the *Streptococcus mutans* more effectively compared to the culture filtrate obtained by culturing in the MRS medium.

Although the embodiments of the present invention have been described in detail above, it will be obvious to those skilled in the art that the scope of the present invention is not limited to these embodiments and that various modifications and changes are possible without departing from the technical spirit of the present invention as defined in the appended claims.

### Industrial Applicability

The present invention relates to an antibacterial composition, and more particularly, to an antibacterial composition comprising a culture filtrate of a Lactobacillus-based strain capable of effectively killing harmful bacteria such as the causative bacteria of periodontitis.

## Claims

1. An antibacterial composition comprising an LP3 culture filtrate obtained by culturing a *Lactobacillus plantarum* in a first medium containing yeast extract and peptone as a nitrogen source(N-source) without containing beef extract, wherein the antibacterial composition has an antibacterial activity against a Streptococcus *mutans* strain.

2. The antibacterial composition of claim 1, wherein a content ratio of the nitrogen source and a carbon source in the first medium is 0.3 to 1: 1.

3. The antibacterial composition of claim 2, wherein a content ratio of the yeast extract and the peptone in the first medium is 0.5 to 4: 1.

4. The antibacterial composition of claim 1, wherein the nitrogen source is contained in 20 to 35 % by weight based on a total weight of the first medium.

5. The antibacterial composition of claim 2, wherein the first medium contains glucose as the carbon source.

6. The antibacterial composition of claim 1, wherein the first medium contains 2 to 4 % by weight of dibasic potassium phosphate, 8 to 10 % by weight of sodium acetate trihydrate, 0.01 to 1 % by weight of magnesium sulfate heptahydrate, 0.01 to 1 % by weight of manganese sulfate tetrahydrate, 3 to 5 % by weight of triammonium citrate, and 1 to 3 % by weight of polysorbate 80, based on a total weight of the first medium.

7. The antibacterial composition of claim 1, wherein the Lactobacillus plantarum is *Lactobacillus plantarum* LP3 (Accession Number: KCTC 10782BP).

8. The antibacterial composition of claim 1, wherein the culturing is performed for 6 to 48 hours at a temperature of 30 to 40 °C.

9. The antibacterial composition of claim 1, wherein the antibacterial composition comprises a LA1 culture filtrate obtained by culturing an *Lactobacillus acidophilus* in a second medium containing the yeast extract and isolated soybean protein as the nitrogen source without containing the beef extract.

10. The antibacterial composition of claim 9, wherein a content ratio of the nitrogen source and a carbon source in the second medium is 0.5 to 1.5: 1.

11. The antibacterial composition of claim 10, wherein a content of the yeast extract and the isolated soybean protein in the second medium is 1 to 10: 1.

12. The antibacterial composition of claim 9, wherein the nitrogen source is contained in 30 to 60 % by weight based on a total weight of the second medium.

13. The antibacterial composition of claim 10, wherein the second medium contains glucose as the carbon source.

14. The antibacterial composition of claim 9, wherein the second medium contains 2 to 4 % by weight of dibasic potassium phosphate, 5 to 10 % by weight of sodium acetate trihydrate, 0.01 to 1 % by weight of magnesium sulfate heptahydrate, 0.01 to 1 % by weight of manganese sulfate tetrahydrate, 2 to 5 % by weight of triammonium citrate, and 1 to 3 % by weight of polysorbate 80, based on a total weight of the second medium.

15. The antibacterial composition of claim 9, wherein the *Lactobacillus acidophilus* is a *Lactobacillus acidophilus* LA1 (Accession Number: KCTC 11906BP).

16. The antibacterial composition of claim 9, wherein the culturing is performed for 6 to 48 hours at a temperature of 30 to 40 °C.

17. The antibacterial composition of claim 9, wherein after culturing the LP3 culture filtrate and the LA1 culture filtrate, a bacterial cell is removed by at least one process of centrifugation and membrane separation.

18. The antibacterial composition of claim 1, wherein the antibacterial comprises one or more selected from the group consisting of propolis, citron extract, bamboo salt extract, coconut extract, spearmint extract, sage extract, green tea extract, grapefruit extract, aloe extract, licorice extract, chamomile extract, plum extract, red ginseng extract, Ulmi cortex extract, cinnamon extract, tea tree extract, lemon extract, and grapefruit seed extract.

19. A pharmaceutical composition for preventing or treating periodontitis comprising the antibacterial composition according to claim 1 as an active ingredient.

20. A pharmaceutical composition for preventing or treating periodontitis comprising the antibacterial composition according to claim 1 as an active ingredient.
